# EUROPEAN PATENT APPLICATION

(11) **EP 3 896 445 A1**
(43) Date of publication of application: **20.10.2021**
(21) Application number: 20170049.9
(22) Date of filing: 17.04.2020
(51) Int. Cl.: G01N 33/18, G01N 27/27, G01N 27/416, C02F 1/00, C02F 9/00

(54) **SYSTEM AND METHOD FOR PERFORMING REDOX POTENTIAL ANALYSIS IN AN AQUEOUS SOLUTION**

(71) Applicant: Uros AG, 8808 Pfäffikon SZ (CH)
(72) Inventor: Rahikainen, Ilkka, 90530 Oulu (FI); Nousiainen, Marko, 90530 Oulu (FI); Hyvärinen, Jyrki, 90530 Oulu (FI)
(74) Representative: Kolster Oy Ab

(57) **Abstract**

A system and a method for redox analysis. Upstream redox potentials are obtained (302) from an upstream sensor apparatus in an upstream flow of an aqueous solution. Upstream chemical parameters are detected (306) based on the upstream redox potentials. Downstream redox potentials are obtained (308) from a downstream sensor apparatus. Each sensor apparatus (120A, 120B) measures redox potentials as a result of one or more reduced or oxidised chemical species in the aqueous solution. Instructions for processing of the downstream redox potentials are generated (314) based on the detected upstream chemical parameter. The generated instructions instructing to test whether the downstream redox potentials indicate a successive downstream chemical parameter, wherein the successive downstream chemical parameter has evolved in a known chemical manner from the detected upstream chemical parameter. The one or more downstream redox potentials are processed (320) based on the generated instructions.

## Description

### FIELD

Various embodiments relate to a system for performing a redox potential analysis, and a method for performing a redox potential analysis.

### BACKGROUND

Redox potential (also known as reduction/oxidation potential) is a measure (measured in volts) of the tendency of a chemical species to acquire electrons from an electrode or lose electrons to an electrode, i.e., the chemical species is thereby reduced or oxidised.

### BRIEF DESCRIPTION

According to an aspect, there is provided subject matter of independent claims. Dependent claims define some embodiments.

One or more examples of implementations are set forth in more detail in the accompanying drawings and the description of embodiments.

### LIST OF DRAWINGS

Some embodiments will now be described with reference to the accompanying drawings, in which
FIG. 1, FIG. 2A and FIG. 2B illustrate embodiments of a a system for performing a redox potential analysis; and
FIG. 3A and FIG. 3B illustrate embodiments of a method for performing a redox potential analysis.

### DESCRIPTION OF EMBODIMENTS

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

Reference numbers both in the description of the embodiments and in the claims serve to illustrate the embodiments with reference to the drawings, without limiting it to these examples only.

The embodiments and features, if any, disclosed in the following description that do not fall under the scope of the independent claims are to be interpreted as examples useful for understanding various embodiments of the invention.

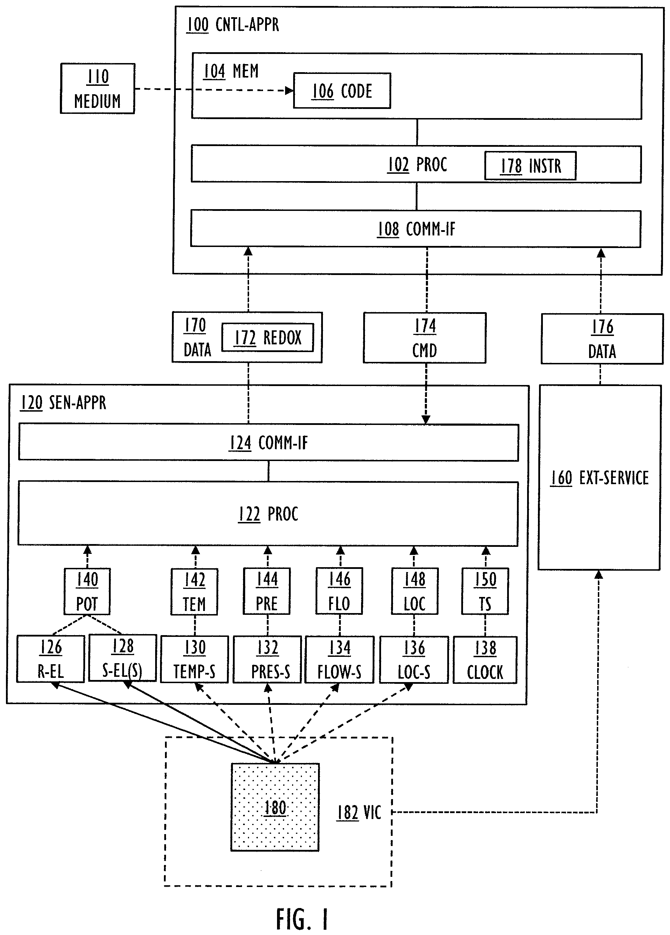

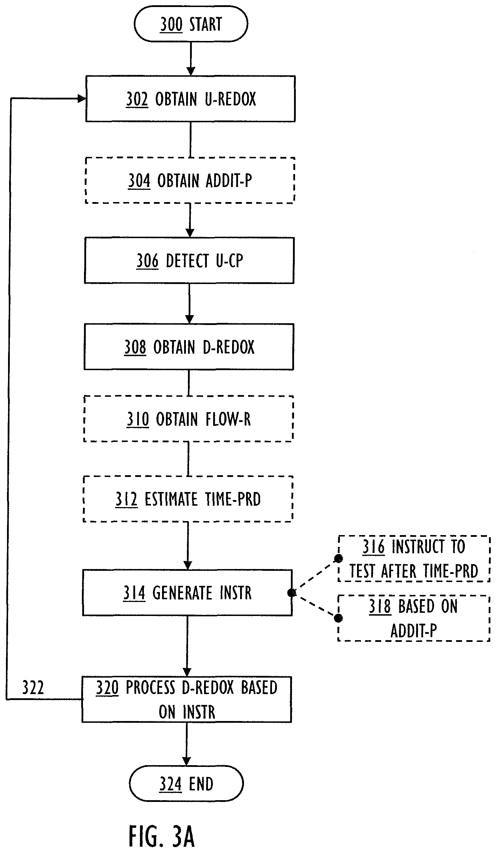

Let us study simultaneously both FIG. 1, which illustrates embodiments of a system for performing a redox potential analysis, and FIG. 3A, which illustrates embodiments of a method for performing a redox potential analysis.

The system comprises a plurality of sensor apparatuses 120 and a control apparatus 100.

Each sensor apparatus 120 comprises comprises a reference electrode 126 and one or more sensing electrodes 128 to measure one or more redox potentials 172 as a result of one or more reduced or oxidized chemical species 212, 214, 216 in an aqueous solution 180.

The aqueous solution 180 is a solution in which the solvent is water, i.e., the one or more reduced or oxidized species 212, 214, 216 are dissolved in the water. The chemical species 212, 214, 216 may be atoms, molecules, molecular fragments, ions, etc., which are subjected to a reduction or oxidation process. For example, when molecules of NaCl (natrium chloride) dissolve in water, there is no NaCl as such in the aqueous solution 180, but Na+ and Cl- ions. Different chemical reactions are possible in the flow of the aqueous solution 180 due to different reactive elements such as oxygen, lack of oxygen, extra electrons or lack of electrons, hydrogen, etc.

The redox potential is a measure of the tendency of the aqueous solution 180 to either gain or lose electrons when it is subjected to change by introduction of a new chemical species 212, 214, 216. The aqueous solution 180 with a higher (more positive) reduction potential than the new chemical species 212, 214, 216 will have a tendency to gain electrons from the new species 212, 214, 216 (i.e. to be reduced by oxidizing the new species), whereas the aqueous solution 180 with a lower (more negative) reduction potential will have a tendency to lose electrons to the new chemical species 212, 214, 216 (i.e. to be oxidized by reducing the new species).

Reduction potentials of the aqueous solution 180 are determined by measuring the potential difference between the sensing electrode 128 in contact with the aqueous solution 180 and the reference electrode 126 connected to the aqueous solution 180.

The reference electrode 126 (made of silver chloride, platinum or gold, for example) is the reference from which the one or more redox potentials 172 are measured. The sensing electrodes 128 are made of different materials (such as platinum, gold, graphite, steel, copper, lithium or carbon, for example) so that different redox potentials 172 are measured from the aqueous solution 180. In effect, the measured redox potentials 172 depend on the chemical species 212, 214 216, which are present, or which are not present in the aqueous solution 180.

US patent 7,108,774 B1, incorporated herein by reference in all those jurisdictions were applicable, discloses redox potential measurement.

Each sensing electrode 128 may be made of a different material so that each electrode produces a different electrode potential in the aqueous solution 180. The potential of each electrode 128 depends on the environment (mostly chemical and electrical properties of the aqueous solution 180) in which they are set.

The measurement principle may be based on an oxidation-reduction reaction on the surface of the electrode 128 in the aqueous solution 180. The electrode materials may be gold, silver, copper, platinum, etc. The electrode potentials of different metals are constant and known in a pure and neutral water at 25 degrees Celsius. For example, Au: 1.5 V, Ag: 0.8 V, Cu: 0.34 V, Al: -1.66 V, Li: - 3.05 V.

When the aqueous solution 180 gets impurities mixed and/or dissolved or it is a mixture of changing chemical components, the corresponding potentials on the electrodes 128 change. The detection principle is based on the measuring and following the potentials online and recognizing the changes. The detection may also be based on the amplitude of the change as well. The detection may also consider a reaction period measured using a time signal 150 from a clock circuit 138 of the sensor apparatus 120.

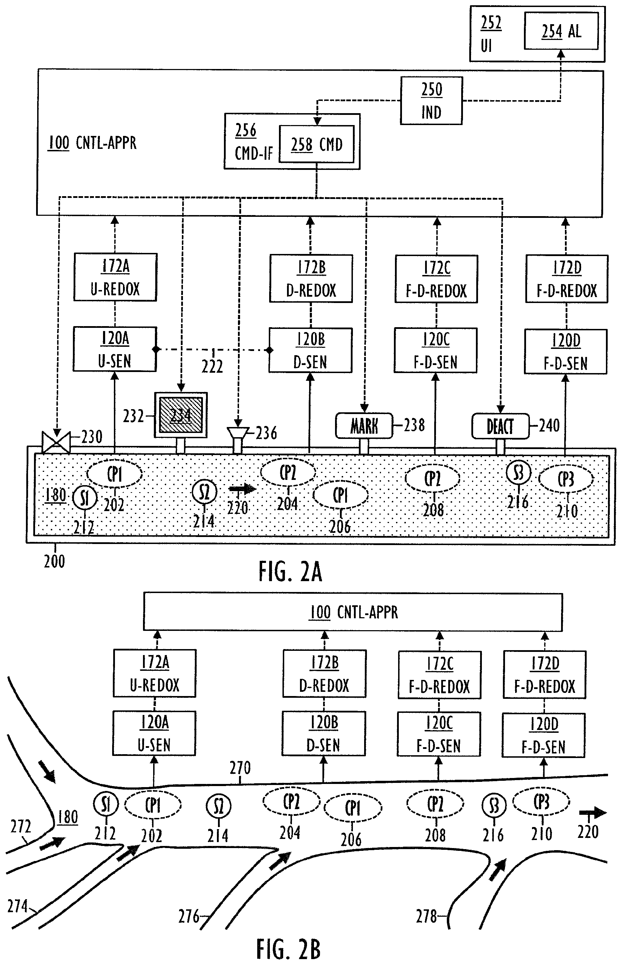

As illustrated in an embodiment of FIG. 2A, the aqueous solution 180 may flow in a pipeline 200, and the sensor apparatuses 120A, 120B, 120C, 120D may be placed along the pipeline 200. The aqueous solution 180 flows in the pipeline in the direction of an arrow 220. The following naming convention for the sensor apparatuses 120 is used: an upstream sensor apparatus 120A is placeable in an upstream flow of an aqueous solution 180, and a downstream sensor apparatus 120B is placeable in a downstream flow of the aqueous solution 180. As shown in FIG. 2A, further downstream sensor apparatuses 120C, 120D may be placed along the downstream flow of the aqueous solution 180. In an embodiment, the pipeline 200 transports freshwater from a natural water source (such as from a groundwater area, lake or river) or from an artificial water reservoir (such as an artificial lake).

As illustrated in an embodiment of FIG. 2B, the aqueous solution 180 may flow along a waterflow (such as a river), which may have a mainstem 270 and a plurality of tributaries 272, 274, 276, 278.

Each sensor apparatus 120A, 120B comprises a processor 122 and a communication interface 124. Each sensor apparatus 120A, 120B may also comprise other parts such as battery to provide electric energy for its operation (or other power source or power interface) and a housing protecting electronics of the sensor apparatus 120A, 120B from external influences (dust, moisture, mechanical shocks, etc.). The battery may be an electric battery converting stored chemical energy into electrical energy. The electric battery may be rechargeable.

The control apparatus 100 comprises a communication interface 108 to communicate with the upstream sensor apparatus 120A and the downstream sensor apparatus 120B, and one or more processors 102, coupled with the communication interface 108, to cause the control apparatus 100 to perform the method for performing the redox potential analysis.

The term 'processor' 102 refers to a device that is capable of processing data. Depending on the processing power needed, the control apparatus 100 may comprise several processors 102 such as parallel processors, a multicore processor, or a computing environment that simultaneously utilizes resources from several physical computer units (sometimes these are referred as cloud, fog or virtualized computing environments). When designing the implementation of the processor 102, a person skilled in the art will consider the requirements set for the size and power consumption of the control apparatus 100, the necessary processing capacity, production costs, and production volumes, for example.

The one or more processors 102 of the control apparatus 100 may be implemented with one or more microprocessors 102, and one or more memories 104 including computer program code 106. The one or more memories 104 and the computer program code 106 are configured to, with the one or more processors 102, cause performance of the data processing operations of the control apparatus 100.

A non-exhaustive list of implementation techniques for the processor 102 and the memory 104 includes, but is not limited to: logic components, standard integrated circuits, application-specific integrated circuits (ASIC), system-on-a-chip (SoC), application-specific standard products (ASSP), microprocessors, microcontrollers, digital signal processors, special-purpose computer chips, field-programmable gate arrays (FPGA), and other suitable electronics structures.

The term 'memory' 104 refers to a device that is capable of storing data run-time (= working memory) or permanently (= non-volatile memory). The working memory and the non-volatile memory may be implemented by a random-access memory (RAM), dynamic RAM (DRAM), static RAM (SRAM), a flash memory, a solid state disk (SSD), PROM (programmable read-only memory), a suitable semiconductor, or any other means of implementing an electrical computer memory.

The computer program code 106 may be implemented by software. In an embodiment, the software may be written by a suitable programming language, and the resulting executable code may be stored in the memory 104 and run by the processor 102.

An embodiment provides a computer-readable medium 110 storing computer program code 106, which, when loaded into the one or more processors 102 and executed by one or more processors 102, causes the one or more processors 102 to perform the computer-implemented method method for performing the redox potential analysis, which will be explained with reference to FIG. 3A. The computer-readable medium 110 may comprise at least the following: any entity or device capable of carrying the computer program code 106 to the one or more processors 102, a record medium, a computer memory, a read-only memory, an electrical carrier signal, a telecommunications signal, and a software distribution medium. In some jurisdictions, depending on the legislation and the patent practice, the computer-readable medium 110 may not be the telecommunications signal. In an embodiment, the computer-readable medium 110 may be a computer-readable storage medium. In an embodiment, the computer-readable medium 110 may be a non-transitory computer-readable storage medium.

Note that the one or more processors 122 of the sensor apparatuses 120A, 120B may be implemented with similar technologies.

In an embodiment, the control apparatus 100 may be a stand-alone control apparatus 100 as in FIG. 1, i.e., the control apparatus 100 is a separate unit as well as the sensor apparatuses 120A, 120B are separate units. The communication interfaces 108, 124 may be implemented with a standard or proprietary wireless or wired communication technology.

The control apparatus 100 as the separate unit may be implemented as a physical unit, or as a service implemented by a networked server apparatus.

The control apparatus 100 may be a computer, laptop computer, tablet computer, phablet, mobile phone, smartphone, general-purpose mobile computing device, or some other electronic data processing apparatus. The control apparatus 100 may be a general-purpose off-the-shelf computing device, as opposed to a purpose-build proprietary equipment, whereby research & development costs will be lower as only the special-purpose software (and not the hardware) needs to be designed, implemented and tested.

The networked server apparatus 100 may be a networked computer server, which interoperates with the fluid sensor apparatus 120 according to a client-server architecture, a cloud computing architecture, a peer-to-peer system, or another applicable distributed computing architecture.

In an embodiment, one or both communication interfaces 108, 124 comprise one or more wireless transceivers operating using one or more of the following: a cellular radio network, a wireless local area network (WLAN), a short-range radio network (such as Bluetooth), a radio network employing a subscriber identity module (SIM), one or more subscriber identity modules selected from among a plurality of subscriber identity modules coupled with the one or more wireless transceivers.

The wireless data transmission may be implemented with a suitable cellular communication technology such as GSM, GPRS, EGPRS, WCDMA, UMTS, 3GPP, IMT, LTE, LTE-A, 3G, 4G, 5G etc. and/or with a suitable non-cellular communication technology such as Bluetooth, Bluetooth Low Energy, Wi-Fi, WLAN, Zigbee, etc. Other applicable technologies include LPWAN (Low Power Wide Area Network), NB-IoT (Narrowband loT), Sigfox, LoRaWAN (Long Range Wide Area Network), QT network, etc. The wired data transmission may be implemented with a suitable standard or proprietary communication technology, such as Ethernet.

However, in an alternative embodiment, the control apparatus 100 and one of the sensor apparatuses 120A, 120B form an integrated apparatus. The integrated apparatus provides a ready-to-use solution that may be installed in a required location, and it only requires a battery, or some other internal/external power source in order to operate. A part of the communication interfaces 108, 124 may then be implemented internally with a standard or proprietary communication bus or a software interface, for example. Also, the processing may be performed with common one or more processors of the integrated apparatus, i.e., the processors 102, 122 belong to a common processing resource pool.

The method starts in 300 and ends in 324. Note that the method may run as long as required (after the start-up of the control apparatus 100 until switching off) by looping from operation 322 back to 302.

The operations are not strictly in chronological order in FIG. 3A, and some of the operations may be performed simultaneously or in an order differing from the given ones. Other functions may also be executed between the operations or within the operations and other data exchanged between the operations. Some of the operations or part of the operations may also be left out or replaced by a corresponding operation or part of the operation. It should be noted that no special order of operations is required, except where necessary due to the logical requirements for the processing order.

In 302, one or more upstream redox potentials 172A are obtained from the upstream sensor apparatus 120A.

In 306, an upstream chemical parameter 202 is detected based on the one or more upstream redox potentials 172A.

In 308, one or more downstream redox potentials 172B are obtained from the downstream sensor apparatus 120B.

In 314, instructions 178 for processing of the downstream redox potentials 172B are generated based on the detected upstream chemical parameter 202. The generated instructions 178 instruct to test whether the one or more downstream redox potentials 172B indicate a successive downstream chemical parameter 204, wherein the successive downstream chemical parameter 204 has evolved in a known chemical manner from the detected upstream chemical parameter 202.

In 320, the one or more downstream redox potentials 172B are processed based on the generated instructions 178.

The described sequence 302-306-308-314-320 describes a way to set the sensor apparatuses 120A, 120B, 120C, 120D along the pipeline 200 or waterflow to form a series of sensing points along the flow 220.

In an embodiment, the upstream chemical parameter 202 and the successive downstream chemical parameter 204 indicate two chemical reactions, products, reduction states or oxidation states in a known series of reduction and/or oxidation processes in the aqueous solution 180.

The progress in the known series will indicate how fast and where the critical processes will happen. This will enhance monitoring accuracy and reliability, and transform decision making quick and reactive to a forecast, whereby critical situations may be prevented quickly and reliably, or an appropriate reaction may be initiated.

So, the upstream sensor apparatus 120A is monitoring the upstream chemical parameter 202 (or, in some cases, also the successive downstream chemical parameter 204). If the upstream chemical parameter 202 is detected, the system will monitor and expect to detect the successive downstream chemical parameter 204 by the downstream sensor apparatus 120B. As shown in FIG. 2A and FIG. 2B, there may be additional downstream sensor apparatuses 120C, 120D to detect the successive downstream chemical parameter 208 and also additional successive downstream chemical parameters 210.

In some reaction series one reaction may be skipped due to a very short reaction time or due to a reaction condition, temperature, ingredients, pressure, light, etc. The additional downstream sensor apparatus 120C, 120D may detect the additional successive downstream chemical parameter 210 even if the successive downstream chemical parameter 204 has not been detected by the downstream sensor apparatus 120B.

In an embodiment, the upstream chemical parameter 202 and the successive downstream chemical parameter 204 relate to a chemical reaction produced by a micro-organism in the aqueous solution 180.

As a first example, a dissimilatory sulfate reduction is given. It is a form of anaerobic respiration that uses sulfate as the terminal electron acceptor. This metabolism is found in some types of bacteria and archaea, which are often termed sulfate-reducing organisms. First, sulfate is activated. This is done by the enzyme ATP-sulfurylase, which uses ATP and sulfate to create adenosine 5'-phosphosulfate (APS). APS is subsequently reduced to sulfite and AMP. Sulfite is then further reduced to sulfide, while AMP is turned into ADP using another molecule of ATP. The reaction steps can be written:
- SO₄²⁻ → (APS →) SO₃²⁻ → H₂S, or
- Sulfate -> Sulfite -> Sulfide.

In this example, the upstream chemical parameter 202 relates to sulfate, the successive downstream chemical parameter 204 relates to sulfite, and the additional successive downstream chemical parameter 210 relates to sulfide.

As a second example an arsenate reduction (in six phases) is given:
- Cys80 and Cys82 form a disulphide bond and the As(V)-enzyme intermediate is formed,
- then attacked by glutathione to form a cysteinyl-AS(V)-glutathione intermediate.
- Arsenate is reduced and arsenite released; a disulphide forms between Cys8 and glutathione.
- A rearrangement transfers glutathione from Cys8 to Cys80 or Cys82.
- Glutaredoxin attacks the disulphide forming Grx-S-S-G.
- A rearrangement restores active site Cys8 concomitant with the Cys80-Cys82 disulphide.

As a third example cyanite and its organic derivatives reaction is given:
- RX + CN⁻ → RCN + X⁻ (nucleophilic substitution), followed by:
   - RCN + 2 H₂O → RCOOH + NH₃ (hydrolysis under reflux with mineral acid catalyst), or 2 RCN + LiAlH₄ + (second step) 4 H₂O → 2 RCH₂NH₂ + LiAl(OH)₄ (under reflux in dry ether, followed by addition of H₂O).

In an embodiment illustrated in FIG. 2A and FIG. 3A, the control apparatus 100 is caused to perform:
- in 310, obtaining a flow rate 146 of the aqueous solution 180, and a distance 222 along the flow of the aqueous solution 180 between the upstream sensor apparatus 120A and the downstream sensor apparatus 120B;
- in 312, estimating a time period for the aqueous solution 180 to flow from the upstream sensor apparatus 120A to the downstream sensor apparatus 120B based on the flow rate 146 and the distance 222; and
- in 314, generating the instructions 178 comprises instructing 316 to test whether the one or more downstream redox potentials 172B indicate the successive downstream chemical parameter 204 after the time period has passed starting from the detection of the detected upstream chemical parameter 202.

With this embodiment, the operations of the control apparatus 100 may be timed and triggered. The time period may be calculated by dividing the distance 222 with the flow rate 146.

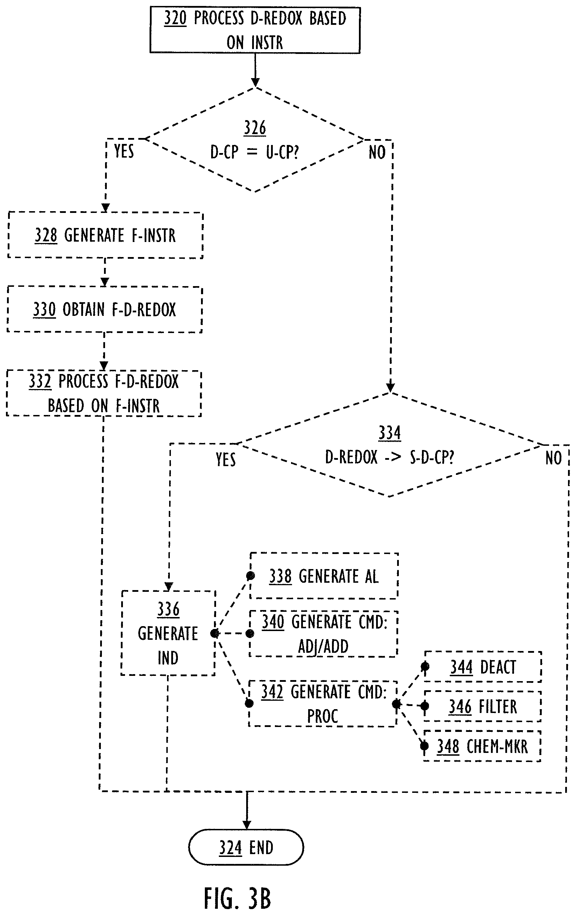

In an embodiment illustrated in FIG. 2A, FIG. 2B and FIG. 3B, the system comprises a further downstream sensor apparatus 120C placeable in a further downstream flow of the aqueous solution 180. The control apparatus 100 is caused to perform:
- in 326 a test is made: if, as a result of the processing 320, the one or more downstream redox potentials 172B indicate a downstream chemical parameter 206, which is similar to the detected upstream chemical parameter 202,
- the YES-branch is entered:
   -- in 328, further instructions to test whether the one or more further downstream redox potentials 172C indicate the successive downstream chemical parameter 208 are generated;
   -- in 330 one or more further downstream redox potentials 172C are obtained from the further downstream sensor apparatus 120C; and
   -- in 332, the one or more further downstream redox potentials 120C are processed based on the further generated instructions.

In an embodiment illustrated in FIG. 3B, the control apparatus 100 is caused to perform:
- in 334 a test is made: if, as a result of the processing, the one or more downstream redox potentials 170B indicate the successive downstream chemical parameter 204, the YES-branch is entered:
   -- in 336, an indication 250 is generated.

As earlier explained, the detection of the upstream chemical parameter 202 and the successive downstream chemical parameter 204 indicate that the known series of reduction and/or oxidation processes in the aqueous solution 180 is progressing, i.e., some (usually unwanted) chemical process is advancing. Based on the indication, various measures may be activated by the control apparatus 100. Let us next study these with reference to FIG. 2A and FIG. 3B.

In an embodiment 338, an alarm 254 is generated in a user interface 252 based on the indication 250. Note that the user interface 252 may be a part of the control apparatus 100, or the user interface 252 may be communicatively coupled with the control apparatus 100. The user interface 252 may thus be a part of user apparatus carried by a person on call.

In an embodiment, the control apparatus 100 comprises a command interface 256.

In an embodiment, the control apparatus 100 is caused to generate 340 a command 258 through the command interface 256 to adjust 230 the flow of the aqueous solution 180 and/or to add an extra flow 234 to be mixed with the aqueous 180 solution based on the indication 250. The command interface 256 may operate according to the previously mentioned standard/proprietary wireless or wired communication technologies. The flow may be adjusted with one or more valves 230 in the pipeline 200 or other types of flow control gear in the pipeline 200 or in the waterflow. By opening or closing the one or more valves 230, the flow may be regulated, blocked or diverted. The extra flow 234 may originate from a tank 232 or other static or dynamic source of water and/or chemicals.

In an embodiment, the control apparatus 100 is caused to generate 342 a command 258 through the command interface 256 to process the aqueous solution 180 based on the indication 250.

In an embodiment, generating 342 the command 258 comprises generating 344 a command through the command interface 256 to begin a deactivation process 240 of a toxic chemical agent in the aqueous solution 180. The deactivation may be performed by mixing a passivation agent into the aqueous solution 180, or by starting a detoxification process.

In an embodiment, generating 342 the command 258 comprises generating 346 a command through the command interface 256 to filter 236 the aqueous solution 180. The filtering 236 may be actuated by directing the flow through a chemical or mechanical filter.

In an embodiment, generating 342 the command 258 comprises generating 348 a command through the command interface 256 to add a chemical marker 238 to the aqueous solution 180.

In an embodiment illustrated in FIG. 1 and 2A, the control apparatus 100 is caused to obtain 304 one or more additional parameters from the upstream sensor apparatus 120A, and/or the downstream sensor apparatus 120B, and/or an external service 160. The external service 160 may be implemented by a networked server. The external service 160 may collect data from public loT (Internet of Things) sensors or from some other data provider.

The additional parameters may indicate one or more of the following:
- a temperature 142 of the aqueous solution 180,
- a pressure 144 of the aqueous solution 180,
- a rate 146 of flow of the aqueous solution 180,
- an illuminance in the vicinity 182 of the flow of the aqueous solution 180,
- an outdoor temperature in the vicinity 182 of the flow of the aqueous solution 180,
- an outdoor air pressure in the vicinity 182 of the flow of the aqueous solution 180,
- an outdoor humidity in the vicinity 182 of the flow of the aqueous solution 180,
- a weather condition in the vicinity 182 of the flow of the aqueous solution 180.

With this embodiment, generating the instructions 178 to test whether the one or more downstream redox potentials 172B indicate the successive downstream chemical parameter 204 in 314 are based on the detected one or more upstream chemical parameters 202, and further based 318 on the one or more additional parameters. These additional parameters may give further evidence whether the one or more downstream redox potentials 172B indicate the successive downstream chemical parameter 204.

In general, the additional parameters may be generated by various sensors 130, 132, 134, 136 in the sensor apparatus 120B, and/or by the external service 160 collecting sensor data or more general data such as weather forecasts. Each sensor may be a converter that measures a physical quantity and converts it into an electrical signal. Such physical quantities may relate to temperature, humidity, speed, pH, acceleration, orientation, an optical quantity such as a transparency or a scattering or an ultraviolet light content, or some other physical quantity, for example. The sensor may also receive some external data and pass it on or generate some further data on the basis of the external data. The external data may relate to positioning, for example. The external data may include signal transmitted by satellites of a global navigation satellite system (GNSS), and/or location coordinates. The external data may also include signals and/or locations used in an indoor positioning system based on radio signals or other techniques (such as magnetic interferences caused by building structures), for example.

With this embodiment, environmental conditions may be taken into consideration while estimating when and where the upstream chemical parameter 202 and the successive downstream chemical parameter 204 may be detected. The environmental conditions may delay the way the successive downstream chemical parameter can be detected due to the temperature, raining, sunlight, etc.

The control apparatus 100 may have a model or a calculation model describing the way the flow is changing over time due to weather conditions or due to usage of water along the river or leakages of water and other material to the flow along the river.

Even though the invention has been described with reference to one or more embodiments according to the accompanying drawings, it is clear that the invention is not restricted thereto but can be modified in several ways within the scope of the appended claims. All words and expressions should be interpreted broadly, and they are intended to illustrate, not to restrict, the embodiments. It will be obvious to a person skilled in the art that, as technology advances, the inventive concept can be implemented in various ways.

## Claims

1. A system for performing a redox potential analysis, comprising:
an upstream sensor apparatus (120A) placeable in an upstream flow of an aqueous solution (180), and a downstream sensor apparatus (120B) placeable in a downstream flow of the aqueous solution (180), each of the upstream sensor apparatus (120A) and the downstream sensor apparatus (120B) comprising a reference electrode (126) and one or more sensing electrodes (128) to measure one or more redox potentials (172A, 172B) as a result of one or more reduced or oxidized chemical species (212, 214, 216) in the aqueous solution (180); and
a control apparatus (100) comprising a communication interface (108) to communicate with the upstream sensor apparatus (120A) and the downstream sensor apparatus (120B), and one or more processors (102), coupled with the communication interface (108), to cause the control apparatus (100) at least to perform:
obtaining (302) one or more upstream redox potentials (172A) from the upstream sensor apparatus (120A);
detecting (306) an upstream chemical parameter (202) based on the one or more upstream redox potentials (172A);
obtaining (308) one or more downstream redox potentials (172B) from the downstream sensor apparatus (120B);
generating (314) instructions (178) for processing of the downstream redox potentials (172B) based on the detected upstream chemical parameter (202), the generated instructions (178) instructing to test whether the one or more downstream redox potentials (172B) indicate a successive downstream chemical parameter (204), wherein the successive downstream chemical parameter (204) has evolved in a known chemical manner from the detected upstream chemical parameter (202); and
processing (320) the one or more downstream redox potentials (172B) based on the generated instructions (178).

2. The system of claim 1, wherein the upstream chemical parameter (202) and the successive downstream chemical parameter (204) indicate two chemical reactions, products, reduction states or oxidation states in a known series of reduction and/or oxidation processes in the aqueous solution (180).

3. The system of any preceding claim, wherein the control apparatus (100) is caused to perform:
obtaining (310) a flow rate (146) of the aqueous solution (180), and a distance (222) along the flow of the aqueous solution (180) between the upstream sensor apparatus (120A) and the downstream sensor apparatus (120B);
estimating (312) a time period for the aqueous solution (180) to flow from the upstream sensor apparatus (120A) to the downstream sensor apparatus (120B) based on the flow rate (146) and the distance (222); and
generating (314) the instructions (178) comprises instructing (316) to test whether the one or more downstream redox potentials (172B) indicate the successive downstream chemical parameter (204) after the time period has passed starting from the detection of the detected upstream chemical parameter (202).

4. The system of any preceding claim, wherein the system comprises a further downstream sensor apparatus (120C) placeable in a further downstream flow of the aqueous solution (180), and the control apparatus (100) is caused to perform:
if (326-YES), as a result of the processing (320), the one or more downstream redox potentials (172B) indicate a downstream chemical parameter (206), which is similar to the detected upstream chemical parameter (202), generating (328) further instructions to test whether the one or more further downstream redox potentials (172C) indicate the successive downstream chemical parameter (208);
obtaining (330) one or more further downstream redox potentials (172C) from the further downstream sensor apparatus (120C); and
processing (332) the one or more further downstream redox potentials (120C) based on the further generated instructions.

5. The system of any preceding claim, wherein the control apparatus (100) is caused to perform:
if (334-YES), as a result of the processing, the one or more downstream redox potentials (170B) indicate the successive downstream chemical parameter (204), generating (336) an indication (250).

6. The system of claim 5, wherein the control apparatus (100) is caused to perform:
generating (338) an alarm (254) in a user interface (252) based on the indication (250).

7. The system of claim 5 or 6, wherein the control apparatus (100) comprises a command interface (256), and the control apparatus (100) is caused to perform:
generating (340) a command (258) through the command interface (256) to adjust (230) the flow of the aqueous solution (180) and/or to add an extra flow (234) to be mixed with the aqueous (180) solution based on the indication (250).

8. The system of claim 5, 6 or 7, wherein the control apparatus (100) comprises a command interface (256), and the control apparatus (100) is caused to perform:
generating (342) a command (258) through the command interface (256) to process the aqueous solution (180) based on the indication (250).

9. The system of claim 8, wherein the control apparatus (100) is caused to perform:
generating (342) the command (258) comprises generating (344) a command through the command interface (256) to begin a deactivation process (240) of a toxic chemical agent in the aqueous solution (180).

10. The system of claim 8 or 9, wherein the control apparatus (100) is caused to perform:
generating (342) the command (258) comprises generating (346) a command through the command interface (256) to filter (236) the aqueous solution (180).

11. The system of claim 8, 9 or 10, wherein the control apparatus (100) is caused to perform:
generating (342) the command (258) comprises generating (348) a command through the command interface (256) to add a chemical marker (238) to the aqueous solution (180).

12. The system of any preceding claim, wherein the control apparatus (100) is caused to perform:
obtaining (304) one or more additional parameters from the upstream sensor apparatus (120A), and/or the downstream sensor apparatus (120B), and/or an external service (160), the additional parameters indicating one or more of the following: a temperature (142) of the aqueous solution (180), a pressure (144) of the aqueous solution (180), a rate (146) of flow of the aqueous solution (180), an illuminance in the vicinity (182) of the flow of the aqueous solution (180), an outdoor temperature in the vicinity (182) of the flow of the aqueous solution (180), an outdoor air pressure in the vicinity (182) of the flow of the aqueous solution (180), an outdoor humidity in the vicinity (182) of the flow of the aqueous solution (180), a weather condition in the vicinity (182) of the flow of the aqueous solution (180); and
generating (314) the instructions (178) to test whether the one or more downstream redox potentials (172B) indicate the successive downstream chemical parameter (204) are based on the detected one or more upstream chemical parameters (202), and further based (318) on the one or more additional parameters.

13. The control apparatus (100) of any preceding claim 1 to 12.

14. A method for performing a redox potential analysis, comprising:
obtaining (302) one or more upstream redox potentials from an upstream sensor apparatus, wherein the upstream sensor apparatus is placeable in an upstream flow of an aqueous solution;
detecting (306) one or more upstream chemical parameters based on the one or more upstream redox potentials;
obtaining (308) one or more downstream redox potentials from a downstream sensor apparatus, wherein the downstream sensor apparatus is placeable in a downstream flow of the aqueous solution, and wherein each of the upstream sensor apparatus and the downstream sensor apparatus comprise a reference electrode and one or more sensing electrodes to measure one or more redox potentials as a result of one or more reduced or oxidised chemical species in the aqueous solution;
generating (314) instructions for processing of the downstream redox potentials based on the detected upstream chemical parameter, the generated instructions instructing to test whether the one or more downstream redox potentials indicate a successive downstream chemical parameter, wherein the successive downstream chemical parameter has evolved in a known chemical manner from the detected upstream chemical parameter; and
processing (320) the one or more downstream redox potentials based on the generated instructions.
